Europäisches Patentamt

European Patent Office

Office européen des brevets

(11) Veröffentlichungsnummer: **0 189 107**
**A2**

(12) **EUROPÄISCHE PATENTANMELDUNG**

(21) Anmeldenummer: 86100463.8

(22) Anmeldetag: 15.01.86

(51) Int. Cl.⁴: **A61K 37/32** , A61K 37/40 , A61K 47/00

(30) Priorität: 23.01.85 DE 3502099

(43) Veröffentlichungstag der Anmeldung:
30.07.86 Patentblatt 86/31

(84) Benannte Vertragsstaaten:
AT BE CH DE FR GB IT LI LU NL SE

(71) Anmelder: Aderhold, Dieter
Am Nocken 47
D-5883 Kierspe(DE)

(72) Erfinder: Aderhold, Dieter
Am Nocken 47
D-5883 Kierspe(DE)

(74) Vertreter: Dipl.-Ing. H. Hauck Dipl.-Phys. W. Schmitz
Dipl.-Ing. E. Graalfs Dipl.-Ing. W. Wehnert Dr.-Ing.
W. Döring
Kaiser-Wilhelm-Ring 41
D-4000 Düsseldorf 11(DE)

(54) Therapeutisches, peptid- und/oder aminosäurehaltiges Mittel.

(57) Es wird ein therapeutisches Mittel beschrieben, das als
Wirkstoff Aminosäuren, Oligopeptide, Polypeptide oder deren
Derivate enthält und eine zur perkutanen bzw. transdermalen
Anwendung geeignete Darreichungsform besitzt.

EP 0 189 107 A2

Rank Xerox

Therapeutisches, peptid- und/oder aminosäurehaltiges Mittel

Die vorliegende Erfindung betrifft ein therapeutisches, peptid- und/oder aminosäurehaltiges Mittel.

Es ist bekannt, derartige peptid- und/oder aminosäurehaltige Mittel zur spezifischen Behandlung einer Reihe von Krankheiten einzusetzen. So werden beispielsweise die oligopeptiden Serinderivate Azaserin und Chloramphenicol, die ebenso wie das als Prototyp eines Antibiotikums bekannte oligopeptide Penizillin eine statische, degenerative, lytische oder abtötende Wirkung gegenüber Viren, Bakterien, Actinomyceten, Fungi, Algen oder Protozoen besitzen, bei einer Vielzahl von Infektionen verordnet. Ebenso werden von den Aminosäuren abgeleitete Hormone, wie z.B. Thyroxin, das bei Hypothyrease, enthyreote Strama und Thyreoditis injiziert wird, therapeutisch verwendet. Ferner wird noch eine Vielzahl von Peptid- und Proteohormonen zur Behandlung von verschiedenen Krankheiten beschrieben. So befindet sich z.B. das polypeptide adrenocorticotrope Hormon (ACTH) als Suspension zur Behandlung von rheumatischen Krankheitsbildern, neurologischen Affektionen, Haut-, Magen-Darm-, Nierenkrankheiten sowie Erkrankungen der Atemorgane im Handel. Neben diesen beispielhaft aufgeführten oligo- und polypeptidhaltigen Mitteln sind auch solche bekannt, die Aminosäuren als therapeutische Substanz enthalten. So werden bei muskulären Dysfunktionen oder als Supplementstoff bei einer Magenübersäuerung solche Präparate verordnet, die die Aminosäure Glykokoll aufweisen. Diese peptid- und/oder aminosäurehaltigen Mittel werden entweder als Säfte, Lösungen, Tabletten, Dragees sowie Zäpfchen oral bzw. anal verabreicht oder parenteral, subkutan, intramuskulär oder intravenös injiziert.

Bei einer Langzeitbehandlung, einer Dauerindikation und/oder bei empfindlichen Patienten bewirkt eine orale oder anale Applikation eine teilweise physisch und/oder psychisch bedingte Abwehrreaktion, so daß es in vielen Fällen zu einem vorzeitigen, unerwünschten Abbruch der Einnahme der verordneten Medikamente kommt. Ebenso treten vielfach bei einer parenteralen Applikation der o.a. Mittel starke Rötungen und/oder Schmerzen an der Injektionsstelle auf, was z.B. bei wiederholten oder sogar dauernden intravenösen Injektionen dazu führen kann, daß nicht mehr in die üblicherweise gut zugänglichen Venen gespritzt werden kann, sondern stattdessen das Mittel über Tropfinfusion und eine Dauerkanüle appliziert werden muß. Ferner erfordern intravenöse Injektionen in der Regel ausgebildetes Fachpersonal.

Der vorliegenden Erfindung liegt die Aufgabe zugrunde, ein therapeutisches Mittel der angegebenen Art zu schaffen, das einfach zu applizieren ist und das bei einer Langzeit- oder Dauerapplikation keine der vorstehend aufgeführten Nebenwirkungen oder unerwünschten Begleiterscheinungen zeigt.

Diese Aufgabe wird erfindungsgemäß durch ein therapeutisches Mittel mit den kennzeichnenden Merkmalen des Patentanspruchs 1 gelöst.

Die Erfindung basiert auf dem Grundgedanken, die peptid-und/oder aminosäurehaltigen therapeutischen Mittel perkutan bzw. transdermal, d. h. also über die Haut, zu applizieren. Hierbei hat es sich nunmehr überraschend gezeigt, daß die jeweiligen Wirkstoffe der peptid-und/oder aminosäurehaltigen Mittel vom Körper über die Haut resorbiert werden, obwohl die klassische Medizin bisher davon ausging, daß hochmolekulare Pharmaka wegen ihrer Molekülgröße und ihrer elektrischen Ladung nicht die Zellschichten des Oberflächenepithels passieren können, da dieses nur eine gewisse Porengröße und Eigenladung aufweist, die die Permeabilität für derartige Mittel begrenzen.

Vorzugsweise werden die peptid- und/oder aminosäurehaltigen Mittel beispielsweise als Salbe, Creme, Paste, Liniment oder Puder appliziert. Als Salbengrundstoffe zur Herstellung der Salben, Pasten, Cremes oder Linimente können beispielsweise die Vaseline aufweisenden Kohlenwasserstoffgel-Grundlagen, die Triglyceride enthaltenden Lipogel-Grundlagen sowie die anorganische und organische Bestandteile aufweisenden Hydrogel-Grundlagen mit einem hohen Wasseranteil verwendet werden. Durch Zusatz von Wasser und einem geeigneten Emulgator zu den o. a. Grundlagen lassen sich abhängig von dem jeweiligen Emulgatortyp Wasser-in-Öl Emulsionslipogele bzw. Öl-in-Wasser Emulsionshydrogele herstellen.

Werden die peptid- und/oder aminosäurehaltigen Mittel als Puder appliziert, so empfiehlt es sich, als Pudergrundlage an der Haut gut haftende, ungiftige Stoffe, beispielsweise Kreide, Magnesiumkarbonat, Kalk, Stärke oder Lycopodium zu verwenden.

Auch eine Anwendung des erfindungsgemäß ausgebildeten Mittels als Lösung ist möglich. Hierbei kann dieses als ideale oder kollodiale Lösung oder als Emulsion oder Suspension vorliegen, wobei als Lösungsmittel Wasser, Alkohol oder eine Mischung von beiden bevorzugt verwendet wird.

Eine spezielle Ausführungsform des erfindungsgemäßen Mittels zur transdermalen Applikation enthält neben Wasser und einem als Salbengrundstoff üblicherweise verwendeten Triglycerid als Wirkstoff die Aminosäure Glykokoll. Ein derartig zusammengesetztes therapeutisches Mittel wurde erfolgreich bei muskulären Dysfunktionen angewendet, wobei bereits nach mehrmaligem Einreiben der erkrankten Muskulatur eine deutliche Besserung des Krankheitsbildes bzw. eine Heilung auftrat.

Eine weitere Ausführungsform des erfindungsgemäßen Mittels wird als kolloidale Lösung transdermal angewendet und enthält als Wirkstoff das Oligopeptid mit der Formel H--His-Phe-Arg-Trp-OH•HCl und als Depotsubstanz Aluminiumhydroxid. Zur Herstellung einer derartigen kolloidalen Lösung werden etwa 20 ml Äthanol-Wasser (1 : 1), etwa 3 - 5 mg Tetrapeptid und etwa 18 - 22 mg Aluminiumhydroxid verwendet. Eine derartige Lösung kann bei normaler Zimmertemperatur,ggf. lichtgeschützt, aufbewahrt werden und ist über einen Monat hinaus haltbar.

Es wurde nunmehr gefunden, daß sich diese Lösungen in ausgezeichneter Weise zur transdermalen Behandlung von Fehlfunktionen des menschlichen Diencephalon und/oder der Hypophyse und dadurch bedingter Störungen, insbesondere zur Behandlung der Multiplen Sklerose, verwenden lassen. Hierbei wird etwa 1/2 ml auf eine willkürlich ausgewählte Hautfläche eingerieben, wobei es gleichgültig ist, welche Hautfläche dafür ausgewählt wird, da die Lösung perkutan/transdermal resorbiert wird und eine Wirkung im gesamten Körper eintritt. Somit können beispielsweise Unterarm, Ellenbeuge oder Oberschenkel mit der Lösung eingerieben werden. Wird eine Wirkung auf bestimmte Körperteile erstrebt, kann es natürlich sinnvoll sein, diese direkt einzureiben. Auf jeden Fall wird aber der gesamte Körper durch die Applikation der Lösung miterfaßt. Bei der Multiplen Sklerose und anderen Erkrankungen mit cerebralen Beeinträchtigungen ist es wegen der besonderen Nähe zum Rückenmark empfehlenswert, vorzugsweise die Körperpartien im Bereich der Lendenwirbel einzureiben.

Die mit dieser erfindungsgemäßen kolloidalen Lösung durchgeführten Behandlungen über einen Zeitraum bis zu 1 1/2 Jahren haben zu folgenden Ergebnissen geführt:

In keinem Fall hat sich das Krankheitsbild während der Therapie verschlechtert. In einigen Krankheitsfällen wurde, nachdem Verbesserungen des Krankheitsbildes nach wenigen Tagen eingetreten waren, die Therpie nach einigen Wochen beendet, ohne daß Verschlechterungen bekannt geworden sind. In einem Fall, in dem das Krankheitsbild nach anfänglichen Verbesserungen über ein Jahr lang stabilisiert gewesen war, wurde auf ein weiteres Einreiben mit der erfindungsgemäßen Lösung verzichtet. Bereits nach vier Wochen trat eine deutliche Verschlechterung des Krankheitsbildes auf, wobei durch ein erneutes Einreiben der frühere, verbesserte Stand wiederhergestellt werden konnte.

Es ist bemerkenswert, daß Patienten, bei denen die Multiple Sklerose mit Schmerzen verbunden war, bereits nach wenigen Tagen durch Anwendung der erfindungsgemäßen kolloidalen Lösung schmerzfrei waren. Ferner berichteten über 60% der Patienten von einer durch die Behandlung bedingten höheren Leistungsfähigkeit und Beweglichkeit, einem Frischegefühl, einem Nachlassen von Spastik und Müdigkeit, einer Normalisierung des Blutdrucks und einer Verbesserung des Tastempfindens. In einigen Fällen ergab sich ferner eine Verbesserung des Sehens, der Gehfähigkeit, der Sprache sowie ein Nachlassen der Ataxie, eine verbesserte Darmtätigkeit, eine Normalisierung des Tag-Nacht-Rhythmus sowie des hormonalen und psychischen Gleichgewichts.

Darüberhinaus scheint der erfindungsgemäßen Lösung noch weitere pharmakologische Bedeutung zuzukommen. So berichteten 60% der behandelten Patienten mit Rheumatismus über Erfolge im Sinne eines deutlichen Nachlassens bzw. Verschwindens der Schmerzen und verbesserter Beweglichkeit und Kräftigung. Ebenso konnte die erfindungsgemäße kolloidale Lösung bei amyotropher Lateralsklerose, schmerzhaften Nervenentzündungen und verschiedenen allergischen Hauterkrankungen, wie z.B. Herpes, Ekzeme, erfolgreich angewendet werden. Unverträglichkeiten und Nebenwirkungen wurden nicht festgestellt. Bei den hier geschilderten Verbesserungen der jeweiligen Krankheitsbilder handelt es sich um objektive Angaben, da diese durch doppelte Blindversuche abgesichert wurden.

Bei der Anwendung des erfindungsgemäßen therapeutischen Mittels zeigt sich überraschenderweise eine prolongierte Wirkung des Wirkstoffes, so daß in vielen Fällen eine gesamte Tagesdosis appliziert werden kann, was durch eine orale oder anale Indikation bzw. bei einer Injektion nicht möglich ist. Dies wird auf die Depotsubstanz zurückgeführt, die bei der o. a. Ausführungsform Aluminiumhydroxid ist. Hierbei wird das Peptid bzw. die Aminosäure durch das Aluminiumhydroxid nicht nur stabilisiert, sondern auch offensichtlich von diesem, bedingt durch seine große innere und äußere Oberfläche, ad- und absorbiert und nur langsam wieder über einen gewissen Zeitraum desorbiert, so daß auf der behandelten Hautfläche ständig nur eine bestimmte Wirkstoffkonzentration vorhanden ist. Sobald diese durch die Resorption des Körpers absinkt, entsteht zwischen der Haut und dem daran anhaftenden Aluminiumhydroxid ein Konzentrationsgefälle, was zur Folge hat, daß weiterer Wirkstoff aus dem Aluminiumhydroxid an die Haut abgegeben wird. Darüberhinaus scheint das Aluminiumhydroxid den ad- oder absorbierten Wirkstoff vor äußeren Einflüssen zu schützen, so daß ein späteres Baden, Duschen oder Schwimmen keinen Einfluß auf die Wirksamkeit des therapeutischen Mittels besitzt. Da sich Aluminiumhydroxid als hautfreundliche Substanz erwiesen hat, wirft eine perkutane bzw. transdermale Dauerbehandlung mit einem diese Depotsubstanz aufweisenden Mittel keine Verträglichkeitsprobleme auf.

Eine weitere Ausführungsform des erfindungsgemäßen therapeutischen Mittels sieht als Wirkstoff das Polypeptid Melanotropin (MSH) in Verbindung mit einer Depotsubstanz, wie beispielsweise Aluminiumhydroxid oder Siliciumdioxid, vor. Dieses als $\alpha$-Melanotropin, $\beta$-Melanotropin und $\delta$-Melanotropin bekannte Polypeptid weist je nach Herkunft zwischen 13 und 22 Aminosäurereste auf und ist bereits isoliert bzw. synthetisch hergestellt worden. Es wurde bisher, soweit bekannt, noch nicht als therapeutisches Mittel eingesetzt.

Es wurde nunmehr gefunden, daß das erfindungsgemäße MSH-haltige Mittel in ausgezeichneter Weise zur perkutanen bzw. transdermalen Behandlung entzündlicher, degenerativer, extraartikulärer und pararheumatischer Erkrankungen geeignet ist. Ferner kann ein derartiges Mittel zur Therapie von Gicht, Allergien oder zur Behandlung von Neuralgien oder Neuritiden verwendet werden.

Hierbei wird der Wirkstoff zusammen mit der Depotsubstanz, beispielsweise als Salbe, Creme oder Lösung, perkutan oder transdermal appliziert. Dabei ist es im Prinzip gleichgültig, welche Hautfläche für die Applikation ausgewählt wird, da die Wirkstoffe, wie vorstehend geschildert, über die Haut aufgenommen werden und somit eine Wirkung im gesamten Körper eintritt.

Auch kann ein derartiges, MSH-haltiges Mittel zur Therapie der Multiplen Sklerose perkutan bzw. transdermal verwendet werden. Hierbei applizierte man über einen längeren Zeitraum einer Reihe von Patienten dieses Mittel transdermal; 2/3 der Patienten hatten ein subjektives - aber durchaus auch objektivierbares - Gefühl der Frische, höheren Lebensmut und größere Leistungsfähigkeit. Eine Verschlechterung des Krankheitsbildes trat in keinem der Fälle auf, und einige nachweisbare Verbesserungen konnten registriert werden. Bei diesen Patienten war ein starkes Nachlassen der Spastik festzustellen. Bei der transdermalen Anwendung entsprechen etwa 0,3 mg $\alpha$-Melanotropin in ihrer Wirkung etwa 0,1 mg des vorstehend erwähnten Tetrapeptids.

Auch bei dieser Ausführungsform des erfindungsgemäßen MSH-haltigen Mittels war nur eine einmalige tägliche Dosierung notwendig, um die erwünschte Wirkung über den Tag zu erzielen. Dies wird auf die Anwesenheit der Depotsubstanz zurückgeführt, die wie vorstehend ausführlich erläutert, auch hierbei einen stabilisierenden und prolongierenden Einfluß auf den Wirkstoff ausübt. Die in den Poren bzw. auf der Haut abgelagerte Depotsubstanz blättert nach einer gewissen Zeit ab oder wird durch den natürlichen, ständigen Hauterneuerungsvorgang unmerklich abgetragen.

Es läßt daher zusammenfassen, daß das erwähnte Aluminiumhydroxid bzw. ein entsprechendes Gel davon (im Handel als Alu-Gel-S von der Firma Serva erhältlich) sich insbesondere als Depotsubstanz für die folgenden Oligopeptid- und/oder Polypeptidwirkstoffe von perkutan bzw. transdermal zu applizierenden Mitteln eignet:

$\alpha$-MSH, $\beta$-MSH, $\delta$-MSH, bzw. Teilstücke davon; Peptide mit den Aminosäuresequenzen Met-Glu-His-Phe-Arg-Trp, Met-Glu-His-Phe-Arg-Trp-Gly, Met-Gly-His-Phe-Arg-Trp, His-Phe, Phe-Arg, Arg-Trp, Met-Glu-His-Phe-Arg, H-Met-(O$_2$)-Glu-His-Phe-D-Lys-Phe-OH; und/oder die Tetrapeptide H-His-Phe-Arg-Trp-OH • 2AcOH, His-Phe-Arg-Trp, H-His-Phe-Arg-Trp-OH • HCL sowie Oligo- und Polypeptide, die in ihrer Aminosäuresequenz diese Tetrapeptide aufweisen.

Zur transdermalen Anwendung als Wirkstoff zur Behandlung von Zuständen infolge von erhöhten Melanotropinspiegels, insbesondere hormonal bedingten Bluthochdrucks, sind die folgenden Oligo- und Polypeptide mit den Aminosäuresequenzen Pro-Leu-Gly-NH$_2$, Pro-Leu, Pz-Pro-Leu, DNP-Pro-Leu-Gly, Pro-Leu-Gly-NH$_2$ • 1/2 H$_2$O, Z-Pro-Leu-Gly-N$_2$, DNP-Pro-Leu-Gly-Ile-Ala-Gly-Arg-NH$_2$ oder PZ-Pro-Leu-Gly-Pro-D-Arg • 2H$_2$O, in Verbindung mit der obigen Depotsubstanz geeignet.

Die Erfindung betrifft ferner ganz allgemein eine Depotsubstanz für perkutan bzw. transdermal zu applizierende therapeutische Mittel, die beispielsweise als Salbe, Creme, Liniment oder Lösung auf die Haut aufgetragen und dort verrieben werden. Bei dieser Depotsubstanz handelt es sich um Aluminiumhydroxid. Diese therapeutischen Mittel können einen beliebigen Wirkstoff enthalten.

Überraschenderweise zeigte sich, daß derartige therapeutische Mittel eine prolongierende Wirkung besitzen, wenn sie Aluminiumhydroxid als Depotsubstanz enthalten. Hierbei wird angenommen, daß Aluminiumhydroxid wenigstens zum Teil oder auch vollständig die Wirkstoffe der jeweiligen Mittel ad- oder absorbiert und diese über einen bestimmten Zeitraum der Haut und damit dem Körper des behandelten Patienten zuführt, wobei das Aluminiumhydroxid an der Haut bzw. in den Poren der Haut abgelagert wird oder so fest in die Haut einzieht, daß z. B. ein späteres Duschen, Baden oder Schwimmen die Wirkung des Mittels nicht beeinträchtigen. Die in bzw. an dem Aluminiumhydroxid gebundenen Wirkstoffe werden über einen längeren Zeitraum somit derartig dem Körper zugeführt, daß ständig eine bestimmte Wirkstoffkonzentration auf der Haut vorhanden ist. Sobald diese durch die Resorption des Wirkstoffes durch den Körper absinkt, bewirkt das Aluminiumhydroxid eine Desorption der Wirkstoffe, was zu einer Wiederherstellung der ursprünglichen Wirkstoffkonzentration auf der Haut führt. Da sich Aluminiumhydroxid erfindungsgemäß als hautfreundliche Substanz erwiesen hat, wirft eine perkutane bzw. transdermale Dauerbehandlung mit einem diese Depotsubstanz aufweisenden Mittel keine Verträglichkeitsprobleme auf, zumal die abgelagerte Depotsubstanz nach einer gewissen Zeit abblättert oder durch den natürlichen, ständigen Hauterneuerungsvorgang unmerklich abgetragen wird.

Eine derartige Desorption der Wirkstoffe ist um so erstaunlicher, da üblicherweise in der chemischen Verfahrenstechnik an Aluminiumhydroxid ad- oder absorbierte Stoffe nur durch ein starkes Erwärmen oder durch eine Druckverminderung zu desorbieren sind.

Eine bevorzugte Ausführungsform der erfindungsgemäßen Depotsubstanz sieht vor, daß diese ein Aluminiumhydroxid-Gel darstellt. Ein derartiges Gel weist eine noch verbesserte prolongierende Wirkung des Wirkstoffes auf und besitzt zudem den Vorteil, daß es die Herstellung des therapeutischen Mittels erheblich vereinfacht.

Die Indikationsgebiete des erfindungsgemäßen Mittels lassen sich wie folgt zusammenfassen:

Fehlfunktionen des menschlichen Diencephalon und/oder der Hypophyse und dadurch bedingter Störungen, beispielsweise des Blutdrucks, der Blasenfunktion, des hormonalen Gleichgewichts, des Stoffwechsels, der Keimdrüsenfunktion, des Wachstums, der Wärmeregulation, des Wasserhaushalts, des Nervensystems und des psychischen Gleichgewichts, insbesondere Multiple Sklerose;

Neuralgien, Neuritiden, rheumatische Erkrankungen, Folgezustände nach und infolge von Polioinfektionen, Gicht und Lateralsklerose, insbesondere entzündliche rheumatische Erkrankungen, degenerative rheumatische Erkrankungen, extraartikuläre rheumatische Erkrankungen und/oder pararheumatische Erkrankungen;

Hautkrankheiten, Erkrankungen der Schleimhäute sowie Allergien, insbesondere Herpes, Schuppenflechte, Ekzeme und Heuschnupfen.

**Ansprüche**

1. Therapeutisches, peptid- und/oder aminosäurehaltiges Mittel, dadurch gekennzeichnet, daß es eine zur perkutanen bzw. transdermalen Anwendung geeignete Darreichungsform besitzt.

2. Mittel nach Anspruch 1, dadurch gekennzeichnet, daß es eine Salbe, eine Creme, eine Paste, ein Liniment, eine Lösung oder ein Puder ist.

3. Mittel nach Anspruch 1 oder 2, dadurch gekennzeichnet, daß es ein Oligopeptid bzw. ein Derivat davon enthält.

4. Mittel nach einem der vorangehenden Ansprüche, dadurch gekennzeichnet, daß es das Tetrapeptid His - Phe - Arg - Trp enthält.

5. Mittel nach Anspruch 1 oder 2, dadurch gekennzeichnet, daß es ein Polypeptid bzw. ein Derivat davon enthält.

6. Mittel nach Anspruch 1 oder 2, dadurch gekennzeichnet, daß es ein von einer Aminosäure abgeleitetes Hormon enthält.

7. Mittel nach Anspruch 1 oder 2, dadurch gekennzeichnet, daß es ein Peptid-und/oder Proteohormon enthält.

8. Mittel nach Anspruch 6, dadurch gekennzeichnet, daß es Melanotropin (MSH) enthält.

9. Mittel nach Anspruch 7, dadurch gekennzeichnet, daß es das adrenocortitrope Hormon (ACTH) enthält.

10. Mittel nach einem der vorangehenden Ansprüche, dadurch gekennzeichnet, daß es als Depotsubstanz ein Ad- und/oder ein Absorptionsmittel enthält.

11. Mittel nach Anspruch 10, dadurch gekennzeichnet, daß die Depotsubstanz Aluminiumhydroxid, Magnesiumhydroxid, Zinkhydroxid, Bariumhydroxid und/oder ein Oxid von Al, Mg, Zn, Ba ist.

12. Mittel nach Anspruch 10, dadurch gekennzeichnet, daß die Depotsubstanz ein Silikat, ein Magnesium-, Barium- und/oder Calciumsalz und/oder Siliciumdioxid ist.

13. Mittel nach einem der vorangehenden Ansprüche, dadurch gekennzeichnet, daß es eine wässrige, alkoholische kolloidale Lösung mit etwa 1 - 20 mg Peptid und/oder Aminosäure und etwa 10 - 40 mg Depotsubstanz in 20 ml Lösungsmittel ist.

14. Mittel nach Anspruch 1, 2 oder 10 - 12, dadurch gekennzeichnet, daß es eine Aminosäure bzw. ein Derivat davon enthält.

15. Depotsubstanz für perkutan bzw. transdermal zu applizierende therapeutische Mittel, dadurch gekennzeichnet, daß sie aus Aluminiumhydroxid besteht.

16. Depotsubstanz nach Anspruch 15, **dadurch gekennzeichnet**, daß das Aluminiumhydroxid als Gel vorliegt.